(19) Europäisches Patentamt

European Patent Office

Office européen des brevets

(11) **EP 1 882 447 A1**

(12) **EUROPEAN PATENT APPLICATION**

(43) Date of publication:
**30.01.2008 Bulletin 2008/05**

(51) Int Cl.:
*A61B 5/053* (2006.01)     *A61B 5/107* (2006.01)

(21) Application number: **06117762.2**

(22) Date of filing: **24.07.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicants:
• **Nutromnia S.R.L.**
 **00135 Rome (IT)**
• **Calvani, Menotti**
 **00146 Rome (IT)**
• **Granato, Luigi**
 **00157 Roma (IT)**

(72) Inventors:
• **CALVANI, Menotti**
 **00146, Rome (IT)**
• **GRANATO, Luigi**
 **00157, Roma (IT)**
• **MINGRONE, Geltrude**
 **00135, Rome (IT)**

(74) Representative: **Spadaro, Marco et al**
 **Studio Associato LEONE & SPADARO**
 **Viale Europa, 15**
 **00144 Roma (IT)**

(54) **DEVICE FOR THE DETERMINATION OF ANTHROPOMETRIC PARAMETERS AND BODY COMPOSITION**

(57)     The invention discloses an anthropometric unit having means for the determination of body volume (BV) of the subject to be measured selected from the group consisting of optical means, ultrasound means and structured light systems; a bioimpedance system; a system for measuring body weight; a control system and data elaboration unit. The combination of an anthropometric unit and of a bioimpedance system provides reliable measures and the correct determination of the parameters of interest, in particular body volume and body fat. The correct determination of these parameters allows the use of the device of the present invention in the medical field, in particular a correct determination in morbidly obese subjects.

Fig. 1

EP 1 882 447 A1

**Description**

Introduction.

The present invention relates to a device for the determination of anthropometric parameters and body composition. In particular, the present invention relates to a device for use in the determination of body volume, body composition and the application of these measurements in the medical field, for example in the diagnosis and treatment of obesity.

Background Art

Background of the invention

Obesity is an excessive accumulation of body fat. In the young adult, normal levels of body fat are comprised between 12 and 20% of body weight in men and between 20 and 30% of body weight in women, whereas levels larger than 25% of body weight in men and over 33% in women are defined as obesity (BUCCHOLZ, AC, et al. The Validity of Bioelectrical Impedance Models in Clinical Population. Nutr. Clin. Practice. 2004, no.19, p.433-446).

To measure body fat is difficult and expensive, and no accurate method is easily available for routine clinical use. Among several methods to assess body fat, two are available and accessible in clinical practice. These are bioelectrical impedance analysis (BIA) and dual energy X-ray absorptiometry (DXA).

BIA measures total body impedance or sectional body impedance using electrodes positioned on body surface. The method actually measures body water, which is then used in equations to estimate fat-free mass (FFM), and subtracting this value from weight estimates body fat ( PI-SUNYER, FX. Symposium on Body Weight Regulation and Obesity: Metabolic and Clinical Aspects. Proc. Nutr. Soc.. 2000, no.59, p.505-509).

DXA (Eur J Clin Nutr. 1997 Aug;51 (8):495-503. Comparison of body composition methods: a literature analysis. Fogelholm M, van Marken Lichtenbelt) estimated fat content in bone-free lean tissue is derived by the constant attenuation of pure fat ($R_f$= 1.18-1.21) and the attenuation of bone-free lean tissue ($R_l$ = 1.399). Given the constancy of these two values from subject to subject, the ratio of the attenuation at the lower energy relative to the higher energy in soft tissue for the low and high energy x-rays (40 keV and 70 keV) is a function of the proportion of the R values for fat and lean in each pixel.

The limitations of these two techniques in measuring fat accumulation in morbidly obese subjects, i.e. subjects with a body mass index (BMI) > 40 kg/m$^2$, reside in an incorrect estimation of the total body water by BIA and in technical reasons by which morbidly obese subjects cannot accommodate into a DXA, TAC or NMR imaging devices. In fact, these devices are large enough only for normal weight or obese subjects, but not for morbidly obese subjects. Furthermore, an assumption underlying the use of DXA states that measurements are not affected by the anteroposterior thickness of the body. Consistently studies have shown that thickness greater than 25 cm has an effect, violates the assumption, and typically overestimates the fat mass (LASKEY, M.A., et al. The influence of tissue depth and composition on the performance of the Lunar dual-energy X-ray absorptiometer whole-body scanning mode. Eur. J. Clin. Nutr.. 1992, vol. 46, no.1, p.39-45.)

For the average male and female adult weighing 70 kg, water constitutes approximately 75% of body weight, respectively. The total body water (TBW) compartment consists of the extracellular water (ECW) (approximately 45%) and the intracellular water (ICW) (approximately 55%). It has been shown that the ratio of TBW to body weight differs greatly among individuals, and that these differences are mostly attributable to differences in the amount of adipose tissue (MARTINOLI, R, et al. Total Body Water Estimation Using Bioelectrical Impedance. Acta Diabetol.. 2003, no.40, p.S203-S206). As this method measures water, it is inaccurate in individuals with oedema or electrolyte disturbances. Changes in ambient temperature and in the placement of the electrodes can also interfere with the results obtained. A number of different instruments are available for measuring fatness by BIA, and some instruments are much superior than others in accuracy and reliability ( BUCCHOLZ, AC, et al. The Validity of Bioelectrical Impedance Models in Clinical Population. Nutr. Clin. Practice. 2004, no.19, p.433-446).

Not only the total amount of fat carried by an individual is important, but also where the fat is distributed in the body. Studies have shown that fat in a central or upper body (android) distribution is most related to health risk ( SEGAL, KR, et al. Estimation of Human Body Composition by Electrical Imedance Methods: a Comparative Study. J. Appl. Physiol.. 1985, no.58, p.1565-1571).

Although the best and most accurate way to measure central obesity is by magnetic resonance imaging (ALBU, JB, et al. Visceral Fat and Race-dependent Health Risk in Obese Nondiabetic Premenopausal Women. Diabetes. 1997, no.46, p.456-462) or computer-assisted tomography scanning (ABATE, N, et al. Relatioship of Generalized and Regional Adiposity to Insulin Sensitivity in Men with NIDDM. Diabetes. 1996, no.45, p.1684-1693), these approaches are too expensive for routine use and, indeed, they are inadequate to accommodate morbidly obese subjects. As a result, simple anthropometric measurements can be used.

Several factors limit the use of BIA as a valid predictor of the amount of body fat in extremely obese individuals.

The presence of increased body fat may affect the validity of BIA. The validity of BIA decreases with increasing obesity and improves with weight loss, because the abdominal adiposity invalidates the assumption that the body is a cylindrical conduct or (BUCCHOLZ, AC, et al. The Validity of Bioelectrical Impedance Models in Clinical Population. Nutr. Clin. Practice. 2004, no.19, p.433-446). Studies have documented an overestimation of the percentage of FFM by approximately 3.4% of body weight or an underestimation of the percentage of fat mass by 8.2% of the body weight in overweight and obese adults (EISENKOBL, J, et al. Underestimation of Percentage Fat Mass Measured by Bioelectrical Impedance Analysis Compared to Dual Energy X-Ray Absorptiometry Method in Obese Children. Eur. J. Clin. Nutr.. 2001, no.55, p.429-432).

Different Authors (SWAN, PD, et al. Anthropometry and Bioelectrical Impedance Inconsistency Predict Fatness in Women with Regional Adiposity. Med. Sci. Sports Exerc.. 1999, no.31, p.1068-1075) examined the accuracy of several anthropometric and BIA regression equations to estimate the percentage of body fat in women with either upper body or lower body fat distribution patterns.

Some BIA instruments are based on leg-to-leg or hand-to-hand impedance to predict the percentage of fat mass directly. Although attractive and simple to use as alternatives to the whole-body BIA model, the random error resulting from these instruments may be large (BUCCHOLZ, AC, et al. The Validity of Bioelectrical Impedance Models in Clinical Population. Nutr. Clin. Practice. 2004, no.19, p.433-446). Most studies using these methods have focused on overweight and obese individuals to measure the percentage of fat mass. It was found that the measurement of the percentage of fat mass was underestimated in the leg-to-leg model in boys more than in girls with android obesity (DEURENBERG, P, et al. The Validity of Predicted Body Fat Percentage from Body Mass Index and from Impedance in Samples of Five European Populations. Eur. J. Clin. Nutr.. 2001, no.55, p.973-979). In the Martinoli et al. study (see above), a leg-to-leg model overestimated the percentage of fat mass in girls and women, but not in boys and men. The authors noted that hand-to-hand BIA measures the impedance of the upper body only and derives the estimation to the amount of the whole body. In conclusion, equations in leg-to-leg and hand-to-hand BIA models take into account differences in body fat distribution, and their validity remains limited.

Other systems have been proposed in the art. For example GB 2220752 A (CHECKMATE INTERNATIONAL LTD) 17.01.1990 discloses a method for determining a person's density or fat content by positioning this person at a point in the space between a light box and a light intensity measurement device and measuring the light intensity with the person standing in different orientations. The volume of the person is determined together the weight and the density and fat content are calculated thereby.

US 2005/0039763 A (MATTHIAS KRAEMER, PAUL CHAMNEY) 24.02.2005 discloses a method and relative apparatus for determining hydration status of a patient by taking certain measures from various compartments. The method determines an anthropometric measure and intra- and extracellular water volume and thus deriving a conclusion on the hydration status of a patient suffering from renal failure. Information on muscle and fat content can also be derived, thus taking conclusion on nutritional status of the patient.

The state of the art gives no information on devices combining body weight and bio-impedance information with an accurate three-dimensional reconstruction of the human body in order to estimate body composition.

There is still the need of a device for the determination of body composition and other anthropometric parameters which is reliable, not excessively expensive and that can be applied with morbidly obese subjects. Another need is the availability of a non-invasive technique. Additional needs are easy of use, time saving and versatile device.

The present invention solves the above mentioned problems by providing the device disclosed in the following description.


Disclosure of Invention


Summary of the invention


The present invention provides a device which comprises:

1. an anthropometric unit having means for the determination of body volume (BV) of the subject to be measured selected from the group consisting of optical means, ultrasound means and structured light systems;
2. a bioimpedance system;
3. a system for measuring body weight,
4. a control system and data elaboration unit.


The combination of an anthropometric unit and of a bioimpedance system provides reliable measures, thus allowing the correct determination of the parameters of interest, in particular body volume and body fat. The correct determination of these parameters allows the use of the device of the present invention in the medical field. In this way, the person skilled in the art will be able to make correct determinations also in morbidly obese subjects.

The device according to the present invention has also further advantages with respect to the state of the art.
The device is easy to use, not excessively expensive, fast and can be used in the determination of different body parameters, such as total or compartment body volume, body surface area, girt circumference and abdominal circumference and volume; density, fat-free mass and fat mass either total or in different compartment.
The device according to the present invention will be now described in details even by means of Figures.

Brief Description of Drawings

Figure 1 gives a schematic view of the working of the anthropometric unit.
Figure 2 gives a schematic view on how the anthropometric carries out a measurement on a human subject.

Mode(s) for Carrying Out the Invention

Detailed description of the invention
The Anthropometric Unit (briefly AU) is a mechanical structure capable of displacing said means for the determination of body surface of the subject to be measured in the three-dimension space where the subject to be measured is placed.
The Bioimpedance System (briefly BS) is a device measuring body impedance.
The system for measuring body weight (BWS) is a conventional system, such as a scale.
Finally, the control system and data elaboration unit (CS) coordinates and controls the acquisition data processes, elaborates the information and gives the results.

Anthropometric unit

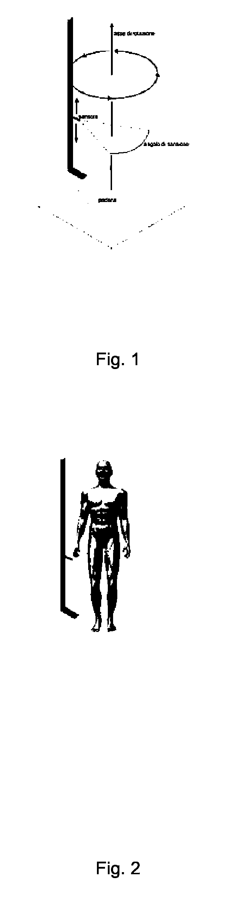

This system comprises a vertical axis carrying the means for the determination of body surface, which can be in the form of a sensor. In a first embodiment, the sensor is an optical sensor. The sensor can be positioned at any height along the vertical axis and it accurately measures the distance from an object inside a certain region of interest. The vertical axis can be of any convenient height, typically 2 m.
As shown in Figure 1, the vertical axis can rotate around a platform, such as a circular basis, where the subject is standing (Figure 2).
With reference to Figures 1 and 2, the AU comprises at least one sensor fixed on a vertical support, for example a pole, translating along a vertical axis while acquiring a series of distance values from the sensor itself and the body to be measured. This vertical scanning is repeated over 360°. To this purpose, any suitable means to rotate the vertical support around the body to be measured is suitable for the present invention. For example, the support, such as a platform, where the subject stands up can rotate while the vertical axis where the sensor system is positioned does not rotate. Alternatively, the subject stands on a fixed platform and the vertical support with the sensor rotates around the subject. The same results can be obtained by using an intelligent system which acquires data in the regions of interest. The software derives through a system of mathematical equations an accurate geometric model of the body. A series of currently used geometrical 3D models, such as those based on triangulation or volumetric methods for integrating range images, are already available (L. De Floriani, E. Puppo. Constrained Delaunay triangulation for multiresolution surface description. Proc. of the 9th Intl. Conf. on Pattern Recognition, pp. 567-569, November 1998; A. Hilton. On reliable surface reconstruction from multiple range images. Technical Report VSSP-TR-5/95, October 1995).
Similarly, the distance measurement can be determined by different commercial sensor systems including the LMS or the RULER produced by SICK (Düsseldorf, Germany). The distance can be measured also by using ultrasound, structured light systems, or other similar systems. At the same manner, there are many commercially available mechanical systems to position the sensor in the space, such as those made by BRIC Engineered System (Oshawa, ON, Canada).

Bioimpedance system and scale

A foot and hand metal plates are used to inject a known electrical current (usually 800 $\mu$A and 50 kHz), while the relative impedance to the flow of the current through the body is measured. The information acquired are impedance and reactance. The Bioimpedance system used in the present invention can be anyone common in current practice, such as for example the commercially available system by Tanita Corp. (Tokyo, Japan).
Total body water (TBW) measured by BIA enters in the following equation to calculate Fat-free mass (FFM):
$$FFM = \frac{TBW}{0.73}$$

The fat mass (FM) is computed as the difference between body weight (BW) and FFM.
$$FM = BW - FFM$$

As currently performed in clinical practice, the extracellular and intracellular fluids can be measured by using multifrequency analysis systems of BIA. Models more accurate and complex than that previously reported can also be applied.

The body weight (which is used in the equation reported above) is measured by a scale suitably positioned at the basis of the device to form a common system. The subject to be measured stands on the scale, which, in an embodiment of the invention can constitute the platform or be integrated in the platform.

Control system and data elaboration unit

The computer system controls the mechanical movements of the device, acquires the measured data and processes them. The most relevant function is data processing and analysis with the final 3D reconstruction of the body, which is a component of a model describing the electrical characteristics of the body. The final product of the model is the data on fat mass and the fat-free amount and distribution. The body volume measured using the sensor system enters the equation below:

$$FM(\%) = \left[ \left( 2.118 \cdot \frac{BV}{BW} \right) - \left( 0.78 \cdot \frac{TBW}{BW} \right) - 1.354 \right] \cdot 100$$

as reported by LOF, M, et al. Hydration of fat-free mass in ealthy women with special reference to the effect of pregnancy. Am. J. Clin. Nutr.. 2004 Oct, vol.80, no.4, p.960-5. In the above equation BV is the body volume and BW is the body weight.

Synthetically, the parameters measured are BW and total body volume or distrectual body volume. While total body water can be measured by either BIA or the sensor system the volume of single body sections can be measured only by the present device. Furthermore, multifrequency BIA allows to measure intracellular water and extracellular water with subsequent calculation of the intracellular and the extracellular compartments. Industrial Applicability

The device according to the present invention can be useful also for other applications, such as for example the measurement of body surface for drug administration based on body surface, district weight reduction, lipodistrophy, aesthetic medicine, reconstruction of limbs to realize tailor made bone or limb prothesis.

**Claims**

1. Device for the determination of anthropometric parameters and body composition of a subject comprising: an anthropometric unit having means for the determination of body volume (BV) of the subject to be measured selected from the group consisting of optical means, ultrasound means and structured light systems; a bioimpedance system; a system for measuring body weight; a control system and data elaboration unit.

2. Device according to claim 1, wherein said anthropometric parameter is selected from the group consisting of total or compartment body volume, body composition, body surface area, girt circumference and abdominal circumference and volume; density, fat-free mass and fat mass either total or in different compartment.

3. Device according to any of claims 1-2, wherein said means are fixed on a vertical support and can move along a vertical axis and said vertical axis can rotate around said subject.

4. Device according to any of claims 1-2, wherein said subject rotates around a vertical axis and said vertical axis is fixed.

5. Device according to claim 4, wherein said subject is standing on a rotatable platform.

6. Device according to any one of claims 1-5, wherein an intelligent system is used to provide a geometrical model of the body of said subject.

7. Method for measuring fatness (FM%) in a subject comprising the steps of: a) determining body volume *BV* of said subject by means of a device comprising a sensor which translates along a vertical axis acquiring a series of distance values from said sensor and said body and repeating said distance value acquisition all around said body, to form a set of distance values and inputting said set of distance values into a computing system calculating said body volume; b) determining total body water *TBW* by means of bioimpedance analysis and inputting said *TBW* into the same computing system of step a); c) determining body weight *BW* and inputting *BW* into the same computing system of step a); making said computing system to calculate fatness according to the following equation:

$$FM(\%) = \left[ \left( 2.118 \cdot \frac{BV}{BW} \right) - \left( 0.78 \cdot \frac{TBW}{BW} \right) - 1.354 \right] \cdot 100$$

wherein: *BV* is Body Volume, BW is Body Weight, *TBW* is Total Body Water.

8. Method according to claim 7, wherein said sensor of step a) is selected from the group consisting of: optical sensor, ultrasound sensor and structured light system sensor.

9. Method according to claim 7 or 9, wherein said bioimpedance analysis is carried out by taking a foot-hand measure.

Fig. 1

Fig. 2

**European Patent Office** | **EUROPEAN SEARCH REPORT** | Application Number

EP 06 11 7762

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/101884 A1 (WEEKS TANYA A S [CA] ET AL) 12 May 2005 (2005-05-12) | 1,2,6 | INV. A61B5/053 A61B5/107 |
| A | * paragraphs [0006], [0021] * | 3-5,7-9 | |
| | ----- | | |
| X | WO 2004/078040 A (CORPUS E AG [DE]; RUTSCHMANN DIRK [DE]) 16 September 2004 (2004-09-16) | 1,2,4-6 | |
| Y | * abstract * | 3 | |
| A | * page 3, paragraph 1 * * page 7, paragraph 1 * | 7-9 | |
| | ----- | | |
| Y | LOF MARIE; FORSUM ELISABET: "Hydration of fat-free mass in healthy women with special reference to the effect of pregnancy" THE AMERICAN JOURNAL OF CLINICAL NUTRITION, vol. 80, no. 4, October 2004 (2004-10), pages 960-965, XP002408081 | 7-9 | |
| A | * page 961 * | 1-6 | |
| | ----- | | |
| Y | US 2001/030754 A1 (SPINA MARIO J [US] ET AL) 18 October 2001 (2001-10-18) | 7-9 | |
| A | * paragraphs [0059], [0070]; figure 1 * | 1-6 | |
| | ----- | | |
| Y | MARTINOLI R; MOHAMED E I; MAIOLO C; CIANCI R; DENOTH F; SALVADORI S; IACOPINO L: "Total body water estimation using bioelectrical impedance: A meta-analysis of the data available in the literature" ACTA DIABETOLOGICA, vol. 40, no. S1, October 2003 (2003-10), pages S203-S206, XP002408082 | 7-9 | |
| A | * abstract * | 1-6 | |
| | ----- | | |
| Y | US 4 406 544 A (TAKADA MUNEKAZU [JP] ET AL) 27 September 1983 (1983-09-27) | 3 | |
| A | * abstract * * column 3, paragraph 2 * | 1,2,4-9 | |
| | ----- | | |

**TECHNICAL FIELDS SEARCHED (IPC)**

A61B

The present search report has been drawn up for all claims

| Place of search | Date of completion of the search | Examiner |
|---|---|---|
| Berlin | 24 January 2007 | Clevorn, Jens |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 06 11 7762

This annex lists the patent family members relating to the patent documents cited in the above-mentioned European search report.
The members are as contained in the European Patent Office EDP file on
The European Patent Office is in no way liable for these particulars which are merely given for the purpose of information.

24-01-2007

| Patent document cited in search report | | Publication date | Patent family member(s) | | Publication date |
|---|---|---|---|---|---|
| US 2005101884 | A1 | 12-05-2005 | NONE | | |
| WO 2004078040 | A | 16-09-2004 | CA | 2518017 A1 | 16-09-2004 |
| | | | DE | 10309788 A1 | 16-09-2004 |
| | | | EP | 1599136 A1 | 30-11-2005 |
| | | | US | 2006140463 A1 | 29-06-2006 |
| US 2001030754 | A1 | 18-10-2001 | NONE | | |
| US 4406544 | A | 27-09-1983 | CA | 1154956 A1 | 11-10-1983 |
| | | | DE | 3104996 A1 | 26-11-1981 |
| | | | FR | 2475884 A1 | 21-08-1981 |
| | | | GB | 2069690 A | 26-08-1981 |
| | | | JP | 56115904 A | 11-09-1981 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- GB 2220752 A

- US 20050039763 A

### Non-patent literature cited in the description

- **BUCCHOLZ, AC et al.** The Validity of Bioelectrical Impedance Models in Clinical Population. *Nutr. Clin. Practice.,* 2004, (19), 433-446
- **PI-SUNYER, FX.** Symposium on Body Weight Regulation and Obesity: Metabolic and Clinical Aspects. *Proc. Nutr. Soc.,* 2000, (59), 505-509
- *Eur J Clin Nutr.,* August 1997, vol. 51 (8), 495-503
- **LASKEY, M.A et al.** The influence of tissue depth and composition on the performance of the Lunar dual-energy X-ray absorptiometer whole-body scanning mode. *Eur. J. Clin. Nutr.,* 1992, vol. 46 (1), 39-45
- **MARTINOLI, R et al.** Total Body Water Estimation Using Bioelectrical Impedance. *Acta Diabetol,* 2003, (40), S203-S206
- **SEGAL, KR et al.** Estimation of Human Body Composition by Electrical Imedance Methods: a Comparative Study. *J. Appl. Physiol,* 1985, (58), 1565-1571
- **ALBU, JB et al.** Visceral Fat and Race-dependent Health Risk in Obese Nondiabetic Premenopausal Women. *Diabetes,* 1997, (46), 456-462
- **ABATE, N et al.** Relatioship of Generalized and Regional Adiposity to Insulin Sensitivity in Men with NID-DM. *Diabetes,* 1996, (45), 1684-1693

- **EISENKOBL, J et al.** Underestimation of Percentage Fat Mass Measured by Bioelectrical Impedance Analysis Compared to Dual Energy X-Ray Absorptiometry Method in Obese Children. *Eur. J. Clin. Nutr,* 2001, (55), 429-432
- **SWAN, PD et al.** Anthropometry and Bioelectrical Impedance Inconsistency Predict Fatness in Women with Regional Adiposity. *Med. Sci. Sports Exerc,* 1999, (31), 1068-1075
- **DEURENBERG, P et al.** The Validity of Predicted Body Fat Percentage from Body Mass Index and from Impedance in Samples of Five European Populations. *Eur. J. Clin. Nutr,* 2001, (55), 973-979
- **L. DE FLORIANI ; E. PUPPO.** Constrained Delaunay triangulation for multiresolution surface description. *Proc. of the 9th Intl. Conf. on Pattern Recognition,* November 1998, 567-569
- **A. HILTON.** On reliable surface reconstruction from multiple range images. *Technical Report VSSP-TR-5/95,* October 1995
- **LOF, M et al.** Hydration of fat-free mass in ealthy women with special reference to the effect of pregnancy. *Am. J. Clin. Nutr,* October 2004, vol. 80 (4), 960-5